Europäisches Patentamt

European Patent Office

Office européen des brevets

(19)

(11) Publication number: **0 327 960**

**A1**

(12) **EUROPEAN PATENT APPLICATION**

(21) Application number: **89101792.3**

(22) Date of filing: **02.02.89**

(51) Int. Cl.⁴: **C12N 15/00 , C12N 9/24 , C12N 5/00 , C12Q 1/42**

(30) Priority: **11.02.88 US 154766**

(43) Date of publication of application:
**16.08.89 Bulletin 89/33**

(84) Designated Contracting States:
**AT BE CH DE FR GB IT LI NL**

(71) Applicant: **F. HOFFMANN-LA ROCHE & CO. Aktiengesellschaft**
**Postfach 3255**
**CH-4002 Basel(CH)**

(72) Inventor: **Berger, Joel Peter**
**9 Fairfax Drive**
**Livingstone N.J. 07039(US)**
Inventor: **Cullen, Bryan Richard**
**3636 Trail 23**
**Durham North Carolina 27707(US)**

(74) Representative: **Mezger, Wolfgang, Dr. et al**
**Grenzacherstrasse 124 Postfach 3255**
**CH-4002 Basel(CH)**

(54) **Secretable forms of alkaline phosphatase.**

(57) Novel gene expression indicator genes are provided which encode secretable alkaline phosphatase proteins. By coupling these genes to various expression control systems, it is possible to readily detect gene expression activity. Methods are also provided that can be carried out quickly, without cell lysis or disruption, to detect such activity.

Figure 2

EP 0 327 960 A1

# Secretable forms of alkaline phosphatase

The present invention relates to indicator or reporter genes which encode secretable forms of alkaline phosphatase, expression vectors containing such genes, cells transformed with such vectors, genetic systems in which gene expression is to be detected, and methods for detecting the secreted enzymes.

A major area of current research in eukaryotic molecular biology is the elucidation of factors which control the level of gene expression at the transcriptional and post-transcriptional levels. One approach that has been used to facilitate this quantitative determination of gene expression is the use of so-called "indicator" or "reporter" genes. These genes encode enzymes or proteins which can be quantitatively assayed. Fusion of these genes to a promoter, enhancer, terminator or non-coding sequence of interest followed by introduction of the construction into eukaryotic cells, then permits the indirect determination of the effect of that fused sequence on the level of gene expression.

Many genes have been used as indicator genes, including E. coli $\beta$-galactosidase [An et al., Mol. Cell. Biol. 2, 1628-1632 (1982)], xanthine-guanine phosphoribosyl transferase [Chu et al. Nucleic Acids Res. 13, 2921-2930 (1985)], galactokinase [Schümperli et al., Proc. Natl. Acad. Sci. USA 79, 257-261 (1982)], interleukin-2 [Cullen, Cell 46, 973-982 (1986)] and thymidine kinase [Searle et al., Mol. Cell. Biol. 5, 1480-1489 (1985)]. All of these genes, however, have disadvantages. For example, there may be high levels of endogeneous enzyme activity, or assays based on the use of the genes may be very cumbersome to carry out.

The indicator gene that is most widely used is the E. coli enzyme, chloramphenicol acetyltransferase (CAT) [Gorman et al., Mol. Cell. Biol. 2, 1044-1051 (1982)]. The major advantage of this enzyme is the lack of any equivalent enzymatic activity in eukaryotic cells, so that no background problems exist. The principal disadvantage is that the assay using this enzyme remains relatively cumbersome to carry out. It normally requires lysis of the cells followed by incubation and extraction steps, and a thin-layer chromatographic step. Furthermore, this assay procedure requires the use of radioactivity and can be fairly expensive to perform in quantity.

Alkaline phosphatase is frequently assayed in diagnostic laboratories because it can be measured in solution by a very simple, fast and inexpensive colorimetric assay. Alkaline phosphatase is normally not secreted from expressing eukaryotic cells. Thus the use of alkaline phosphatase as an indicator gene would, as is true for CAT, require lysis of the cells.

Genetic expression systems in which indicator genes are used are produced through the use of recombinant DNA technology. In the application of current recombinant DNA procedures, specific DNA sequences are inserted into an appropriate DNA vehicle, or vector, to form recombinant DNA molecules that can replicate in host cells. Circular double-stranded DNA molecules called plasmids are frequently used as vectors, and the preparation of such recombinant DNA forms entails the use of restriction endonuclease enzymes that can cleave DNA at specific base sequence sites. Once cuts have been made by a restriction enzyme in a plasmid and in the segment of foreign DNA that is to be inserted, the two DNA molecules may be covalently linked by an enzyme known as a ligase. General methods for the preparation of such recombinant DNA molecules have been described by Cohen et al. [U.S. patent No. 4,237,224], Collins et al. [U.S. patent No. 4,304,863] and Maniatis et al. [Molecular Cloning: A Laboratory Manual, 1982, Cold Spring Harbor Laboratory]. Because they illustrate much of the state of the art, these references are hereby incorporated by reference.

Once prepared, recombinant DNA molecules can be used to produce the product specified by the inserted gene sequence only if a number of conditions are met. Foremost is the requirement that the recombinant molecule be compatible with, and thus capable of autonomous replication in, the host cell. Much recent work has utilized Escherichia coli as a host organism, because it is compatible with a wide range of recombinant plasmids. Depending upon the vector/host cell system used, the recombinant DNA molecule is introduced into the host by transformation, transduction or transfection.

Detection of the presence of recombinant plasmids in host cells may be conveniently achieved through the use of plasmid marker activities, such as antibiotic resistance. Thus, a host bearing a plasmid coding for the production of an ampicillin-degrading enzyme could be selected from unaltered cells by growing the host in a medium containing ampicillin. Further advantage may be taken of antibiotic resistance markers where a plasmid codes for a second antibiotic-degrading activity at a site where the selected restriction endonuclease makes its cut and the foreign gene sequence is inserted. Host cells containing properly recombinant plasmids will then be characterized by resistance to the first antibiotic but sensitivity to the second.

The mere insertion of a recombinant plasmid into a host cell and the isolation of the modified host will

2

not in itself assure that significant amounts of the desired gene product will be produced. For this to occur, the foreign gene sequence must be fused in proper relationship to a signal region in the plasmid for DNA transcription called a promoter. Alternatively, the foreign DNA may carry its own promoter, as long as it is recognized by the host. Whatever its origin, the promoter is a DNA sequence that directs the binding of RNA polymerase and therefore "promotes" the transcription of DNA to messenger RNA (mRNA).

Given strong promotion that can provide large quantities of mRNA, the ultimate production of the desired gene product will be dependent upon the effectiveness of translation from mRNA to protein. This, in turn, is dependent upon the efficiency of ribosomal binding to the mRNA and upon the stability of the mRNA within the host cell. In eukaryotic cells, the factors governing translational efficiency are poorly understood but appear to include a favorable nucleic acid sequence surrounding an AUG codon which initiates translation [Kozak, Cell 44, 283-292 (1986)]. Factors affecting the stability of the mRNA, which are poorly understood in both prokaryotic and eukaryotic cells, are also critical to the amount of protein production that can be obtained.

Most of the work in the recombinant DNA field to the present has focused on the use of bacterial expression systems such as E. coli. Yet, the use of bacterial cells has a number of undesirable aspects. For example, many proteins and polypeptides produced in E. coli accumulate in the periplasmic space. Recovery of these gene products thus requires disruption of the cells, a process which is inefficient and leads to serious purification problems, as the desired product must be purified from the numerous other E. coli cellular constituents. Also, bacteria cannot carry out glycosylation which is needed to complete the synthesis of many interesting gene products or form the specific disulfide bonds which are essential for the proper conformation and biological activity of many eukaryotic proteins.

To overcome these deficiencies in bacterial expression systems, the attention of genetic engineers is increasingly turning to the use of eukaryotic host cells for recombinant DNA, not only to make desirable polypeptides and proteins but to study the control of gene expression as well. Cells such as yeast and mammalian cells can secrete desired gene products into the culture medium and can also carry out essential glycosylation processes. Yet, the use of mammalian cells for recombinant DNA cloning and expression also poses a host of technical obstacles that must be overcome. For example, the endogeneous plasmids that have proven to be so useful in bacteria are not replicated by higher eukaryotic cells. As a result, other approaches must be taken.

One approach has been to use the lower eukaryotic yeast, Saccharomyces cerevisiae, which can be grown and manipulated with the same ease as E. coli. Yeast cloning systems are available, and through the use of such systems the efficient expression in yeast of a human interferon gene has been achieved [Hitzeman et al., Nature 293, 717-722 (1981)]. Interferon genes do not contain introns, however, and it has been found that yeast cells do not correctly transcribe at least one heterologous mammalian gene that does contain introns, viz. the rabbit β-globin gene (Beggs et al., Nature 283, 835-840 (1980)].

In another approach, foreign genes have been inserted into mammalian cells by means of direct uptake. This has been accomplished by calcium phosphate co-precipitation of cloned genes, by which procedure about 1-2% of the cells can generally be induced to take up the DNA. Such a low level of uptake, however, produces only a very low level of expression of the desired gene product. Where mammalian cells can be found which lack the thymidine kinase (tk) gene (tk⁻ cells), better results can be obtained by co-transformation. Tk⁻ cells, which cannot grow in selective HAT (hypoxanthine-aminopterin-thymidine) medium, can regain this lost enzymatic activity by taking up exogenous DNA (such as herpes simplex viral DNA) containing the tk gene through calcium phosphate co-precipitation. Other DNA covalently ligated to the tk DNA or merely mixed with it will also be taken up by the cells and will often be co-expressed [see Scangos et al., Gene 14, 1-10 (1981)].

In a third approach, viral genomes have been used as vectors for the introduction of other genes into mammalian cells, and systems based upon simian virus 40 (SV 40), papilloma-virus and adenovirus genomes have been described [for review see P.W.J. Rigby, Expression of Cloned Genes in Eukaryotic Cells Using Vector Systems Derived from Viral Replicous, in Genetic Engineering, Vol. 3, R. Williamson, ed., Academic Press, New York, pp. 83-141 (1982)]. These systems, however, suffer from the drawback of limited host cell range. Moreover, viral replication in these systems leads to host cell death. The use of retroviral DNA control elements avoids many of the disadvantages of these viral vector systems.

Gorman et al. [Proc. Natl. Acad. Sci. U.S.A. 79, 6777-6781 (1982)] have shown, for example, that the Rous sarcoma virus long terminal repeat (LTR) is a strong promoter that can be introduced into a variety of cells, including CV-1 monkey kidney cells, chicken embryo fibroblasts, Chinese hamster ovary cells, HeLa cells and mouse NIH/3T3 cells by DNA- mediated transfection.

The present invention provides novel secretable human placental alkaline phosphatases, indicator gene DNA sequences coding for these secretable human placental alkaline phosphatases, recombinant expres-

3

sion vectors comprising the indicator genes operatively linked to expression control sequences, mammalian cells containing such expression vectors, and methods for the use of such DNA sequences in gene expression systems.

More particularly, the present invention provides secretable human placental alkaline phosphatases having from 11 to 25 amino acid residues deleted at the carboxyl-terminus (C-terminus) compared to the wild-type human placental alkaline phosphatase. The effect of such deletions is to permit passage of the enzymes through the membranes of mammalian cells containing the expression vectors of the present invention. In a preferred embodiment, the 24 C-terminal amino acid residues are deleted compared to the wild-type human placental alkaline phosphatase.

The indicator genes of the present invention are superior to previously described indicator genes because the secretable enzymes appear in the medium of cells expressing the genes and can be quickly and accurately detected and measured by a simple colorimetric assay. As a consequence, cell disruption or laborious bioassay which are generally required to detect the expression products of other indicator genes are avoided.

The present invention still further provides methods for detecting factors which influence gene expression, comprising:

(a) providing mammalian cells in culture containing a DNA sequence coding for a secretable human placental alkaline phosphatase having from 11 to 25 amino acid residues deleted at the C-terminus compared to the wild-type human placental alkaline phosphatase;

(b) contacting the mammalian cell culture with a factor influencing gene expression, to cause an altered expression of the DNA sequence and the appearance of an altered amount of the secretable human placental alkaline phosphatase in the culture medium; and

(c) measuring the altered amount of the secretable placental alkaline phosphatase in the culture medium.

In an illustrative embodiment, the mammalian cells expressing the secretable human placental alkaline phosphatases are an African green monkey kidney cell line transformed by an origin minus SV40 viral genome (COS cells), which has been transfected with preferred expression vectors pBC12/RSV/SEAP or pBC12/HIV/SEAP. COS cells contain the SV40 large T antigen in their genome [Gluzman, Cell 23, 175-182 (1981)]. The expression of the latter vector is influenced by the trans-acting gene product (tat) of the Human Immunodeficiency Virus-I (HIV-I), which interacts with the HIV-I long terminal repeat (LTR) promoter in the vector to greatly enhance expression of the secretable human placental alkaline phosphatase gene. Such enhanced gene expression is readily detected by simple colorimetric assay of the culture medium, using p-nitrophenyl phosphate as an enzyme substrate.

Production of a secretable human placental alkaline phosphatases of the present invention can be achieved by culturing mammalian cells containing a recombinant vector comprising a DNA sequence coding for a secretable human placental alkaline phosphatase and isolating the secretable human placental alkaline phosphatase from the culture.

The present invention can be more readily understood by reference to the following Description of the Invention and Examples and to the following figures, in which secretable human placental alkaline phosphatase is abbreviated as "SEAP", and:

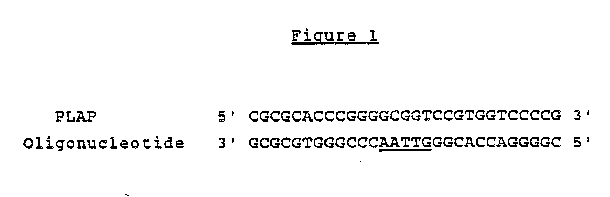

Fig. 1 provides the nucleotide sequence of bases 1510-1539 of the human placental alkaline phosphatase (PLAP) gene and of an oligodeoxynucleotide used to mutate the gene. The five mutated bases are shown underlined;

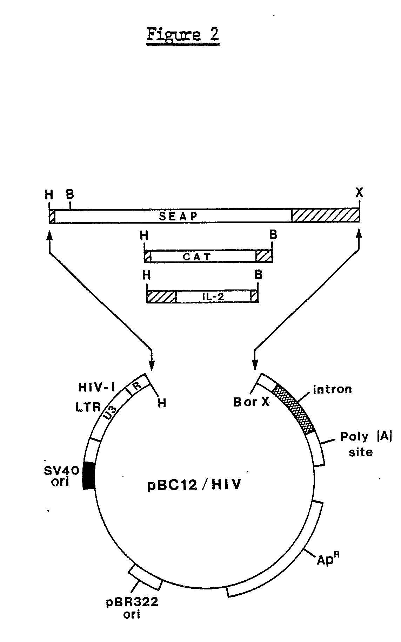

Fig. 2 is a schematic representation of the various pBC12/HIV expression vectors used to compare the use of the SEAP indicator genes with the CAT and IL-2 indicator genes. Each pBC12/HIV construction is based on pXF3/ori, which contains the SV40 origin of replication inserted into pXF3, a poison-sequence minus derivative of pBR322 [Hanahan, J. Mol. Biol. 166, 557-580 (1983); Cullen, supra]. Vector pBC12/HIV also contains a 728 bp HIV-I LTR fragment which includes the entire HIV-I LTR "U3" and trans-activiation responsive regions [Cullen, supra]. A 3' non-coding region derived from the genomic rat preproinsulin II gene [Lomedico et al., Cell 18, 545-558 (1979)] provides a polyadenylation site and a 3' intronic region. The indicator gene cassettes were inserted between the unique vector HindII and BamHI sites as shown. In the case of SEAP, which contains an internal BamHI site, the 3' site was changed to XhoI. The pBC12/RSV vectors are identical except for the substitution of the RSV LTR for the HIV-I LTR. Crosshatched areas denote non-coding regions present in the indicator gene cassette;

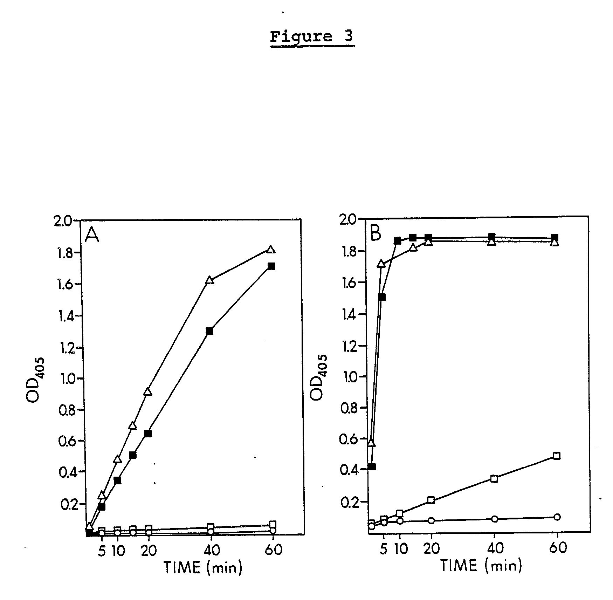

Fig. 3 is a graphical representation of a colorimetric assay of SEAP activity from the media of transfected COS cells, showing changes in absorbance at 405 nm as a function of time. The assay was carried out using 10 $\mu$l (Panel A) and 100 $\mu$l (panel B) of a total of 20 ml of medium from each transfected plate. The figure shows the results obtained with COS cells transfected with plasmids pBC12/HIV/CAT (tat +), control (open circles); pBC12/HIV/SEAP (tat-) (open squares); pBC12/HIV/SEAP (tat +) (closed squares) and pBC12/RSV/SEAP (closed triangles) plates; and

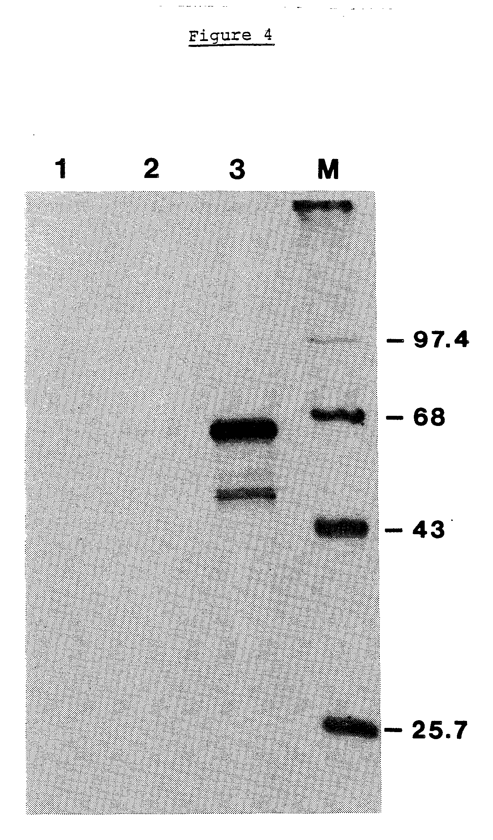

Fig. 4 shows the results of a discontinuous SDS-polyacrylamide gel electrophoretic analysis of the immunoprecipitation of SEAP from lysates of suspensions of transfected COS cells pulse-labeled with [$^{35}$S]-methionine. The figure shows the results obtained with COS cells transfected with plasmids pBC12/HIV/CAT (tat +) (lane 1), pBC12/HIV/SEAP (tat -) (lane 2) and pBC12/HIV/SEAP (tat +) (lane 3). Lane M contained protein molecular weight markers labeled with $^{14}$C (Bethesda Research Laboratories, Gaithersburg, MD). Samples run in lanes 1-3 were isolated by immunoprecipitation using rabbit anti-human placental alkaline phosphatase antibodies and S. aureus protein A, and the gel was developed by autoradiography.

The SEAPs of the present invention are secretable forms of human placental alkaline phosphatase which pass through the membranes of mammalian cells in which they are produced, because they have from 11 to 25 C-terminal amino acid residues deleted compared to the wild-type human placental alkaline phosphatase. The presence of these deleted amino acid residues in the wild-type enzyme causes the enzyme to be anchored to the outer leaflet of the plasma membrane via a phosphatidylinositol-glycan moiety. The complete nucleotide sequence of human placental alkaline phosphatase cDNA and the amino acid sequence predicted therefrom have been disclosed in Kam et al., Proc. Natl. Acad. Sci. USA 82, 8715-8719 (1985).

The present invention also encompasses functional equivalents of secretable human placental phosphatases which contain amino acid substitutions but retain alkaline phosphatase activity and secretability as well as other alkaline phosphatases and equivalents thereof which can be used to measure gene expression activities. Amino acid substitutions in proteins and polypeptides which do not essentially alter biological activity are known in the art and described, e.g., by H.·Neurath and R.L. Hill in "The Proteins", Academic Press, New York (1979), in particular in Fig. 6 of page 14. The most frequently observed amino acid substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and vice versa.

Because the complete DNA sequence of the gene coding for wild-type human placental alkaline phosphatase is known, DNA sequences coding for the SEAPs of the present invention can be chemically synthesized using standard methods known in the art, preferably solid state methods. Alternatively, genomic DNA could serve as a source of the wild-type enzyme. Preferably, the wild-type gene is prepared as cDNA using mRNA from cells actively producing the enzyme as a template, as described in detail below. Suitable deletions of nucleotide residues are then made, preferably by the primer-directed site-specific mutagenesis method of Morinaga et al., Bio/Technology 2, 636-639 (1984). Synthetic oligonucleotides needed for such mutagenesis can be prepared by the phosphoramidite solid support method of Matteucci and Caruthers, J. Am. Chem. Soc. 103, 3185-3191 (1981).

Regardless of its source, DNA encoding the SEAPs can be cloned into bacteria, and the clones can be identified by methods well known in the art. For example, transformants can be selected on the basis of antibiotic resistance properties due to the presence of the vectors used, or complimentary DNA (cDNA) probes could be prepared for the DNA, labeled, and used to detect clones bearing the DNA through the use of hybridization techniques. The DNA can then be excised using an appropriate restriction enzyme or enzymes. E. coli strain MC1061 is the preferred host bacterial cell.

Insertion of the excised DNA into an appropriate expression vector can easily be accomplished when the DNA coding for the SEAPs and the vector have been cut with the same restriction enzyme or enzymes, since complementary DNA termini are thereby produced. If this cannot be accomplished, the single-stranded DNA of the cut ends can be digested back to produce blunt ends, or the same result can be achieved by filling in the single-stranded termini with an appropriate DNA polymerase such as the Klenow fragment of DNA polymerase I. In this way, blunt end ligation into a blunt ended cut vector can be carried out with an enzyme such as T4 DNA ligase.

For insertion of the DNA into an expression vector, any site desired could also be produced by ligating nucleotide sequences (linkers) onto the DNA termini. Such linkers, may comprise specific oligonucleotide sequences that contain restriction site recognition sequences. The cleaved vector and the modified DNA can also be modified by homopolymeric tailing, as described by Morrow in Methods in Enzymology 68, 3-24 (1979).

Expression vectors suitable for use in mammalian cells which could be used in this invention include but are not limited to pBC12MI, pBC12BI, pSV2dhFr, p91023 (B), pcDV1, pRSVcat, pGA291, pGA293, pGA296, pBC12/HIV/IL-2 and pGA300. Vectors pBC12BI and pBC12/HIV/IL-2 are preferred. Such vectors are preferably introduced into suitable mammalian host cells by transfection.

Mammalian host cells that could be used include, e.g., human Hela, H9 and Jurkat cells, mouse NIH3T3 and C127 cells, CV1 African green monkey kidney cells, quail QC1-3 cells, Chinese hamster ovary (CHO) cells, mouse L cells and the COS cell line. The COS cell line is used in an exemplary embodiment of this invention, below.

The SEAP indicator genes of the present invention can be used to detect the activity of any expression vector control elements upstream of the genes. For example, the genes can be used to study the effects of sequence-specific binding proteins that are repressor or activator substances on regulatory systems susceptible to the action of such substances. These proteins can be part of cascade systems; the tat protein is suspected to be such a protein. Furthermore, the genes can be used to detect the effects of other molecules that may interact with and affect the activities of such substances. As used herein, the term "factor influencing gene expression" is intended to encompass all such materials, whether the net effects of their actions upon gene expression are positive or negative.

In an illustrative embodiment of this invention, the factor influencing gene expression is the tat transacting regulating protein which serves to greatly enhance expression of genes under the control of the human immunodeficiency virus (HIV) long terminal repeat (LTR) promoter. Through the use of the indicator genes of the present invention placed downstream of the HIV LTR promoter in a suitable expression vector, it is possible to monitor the effects of drugs, environmental factors and other gene products on the functioning of the promoter and/or the tat gene product or its production. Such a system can be used to screen for agents which might be able to interfere with the production of the tat protein or the function of the promoter, thereby offering a therapeutic approach for the treatment of AIDS. The skilled artisan will immediately recognize numerous systems in which the indicator gene DNA sequences of the present invention can be profitably employed.

Because the SEAPs of the present invention are secreted directly into the culture medium of the producing cells, aliquots of medium can simply be taken for analysis. Any method of alkaline phosphatase assay can be employed. McComb and Bowers, Clin. Chem. 18, 97-104 (1972) disclose optimum buffer conditions for measuring such activity using p-nitrophenylphosphate as a substrate. Another substrate that could be used, e.g., is D-luciferin-O-phosphate, which has been described by Miska et al., J. Clin. Chem. Clin. Biochem. 25, 23-30 (1987).

Preferably, the assay medium is made about 10 mM in L-homoarginine and preheated to about 65°C for about 5 minutes prior to assay. The effect of such treatment is to inhibit any endogeneous phosphatase activities that may be present in the mammalian host cells.

## EXAMPLES

The following non-limiting examples illustrate how a gene encoding a secretable alkaline phosphatase can be constructed and expressed, and how the use of this gene as an indicator gene is superior to other indicator genes in a representative gene expression system. The following general methods were used in these examples.

Unless otherwise specified, percentages given below for solids in solid mixtures, liquids in liquids and solids in liquids are on a w/w ,v/v and w/v basis, respectively.

Small scale isolation of plasmid DNA from saturated overnight cultures was carried out according to the procedure of Birnboim and Doly, Nucleic Acids Res. 7, 1513-1523 (1979). This procedure allows the isolation of a small quantity of DNA from a bacterial culture for analytical purposes. Unless otherwise indicated, larger quantities of plasmid DNA were prepared as described by Clewell et al., J. Bacteriol. 110, 1135-1146 (1972).

Specific restriction enzyme fragments derived by the cleavage of plasmid DNA were isolated by preparative electrophoresis in 1% GTG (Genetic Technology Grade) agarose (Seaplaque, FMC Inc. Rockland, Maine). Five x 7.5 cm gels were run at 50 mA for 1-2 hours in Tris-Borate buffer (Maniatis et al., supra, p. 454) and then stained with 1 μg/ml ethidium bromide to visualize the DNA. Appropriate gel sections were excised and placed in a dialysis bag containing 500 μl of Tris-Borate buffer. The DNA was electroeluted into the Tris-Borate buffer, extracted twice with phenol and three times with chloroform and precipitated at -20°C with ethanol in the presence of 10 μg of tRNA carrier (yeast, Bethesda Research Laboratories, Gaithersburg, MD)

The restriction enzymes, DNA polymerase I (Klenow fragment) and T4 DNA ligase (T4 DNA polymerase) were products of New England Biolabs, Beverly, MA, and the methods and conditions for the use of these enzymes were from the manufacturer's protocol.

For the restriction endonucleases, a unit of activity is defined as the amount of enzyme needed to produce a complete digest of 1.0 μg DNA in 60 minutes in a total reaction volume of 0.05 ml, with digestion carried out at 37°C. The buffer used for all of these enzymes (restriction enzyme buffer) consisted of 100 mM NaCl, 10 mM Tris-HCl (pH 7.5), 5 mM MgCl₂ and 1 mM 2-mercaptoethanol.

T4 DNA ligation was carried out for 16 hours at 4°C in ligation buffer containing 60 mM Tris-HCl (pH 7.5). 10 mM MgCl₂, 10 mM dithiothreitol and 0.1 mM ATP. A unit of T4 DNA ligase activity is defined as the amount required to give 50% ligation of HindIII fragments of lambda DNA in 30 minutes at 16°C in 20 μl of incubation mixture and a 5' DNA termini concentration of 0.12 μM (300 μg/ml).

Klenow blunt-ending of single-stranded DNA ends was carried out in restriction enzyme buffer which had been adjusted to contain 1 mM of each of dGTP, dATP, dCTP and dTTP. A unit of activity is defined as the amount converting 10 nmoles of deoxyribonucleotides to an acid insoluble form in 30 minutes at 37°C.

Culture media used included Iscove's Modified Dulbecco's Medium (IMDM) from Grand Island Biological Co. (GIBCO), Grand Island, NY, and Luria Broth (LB), containing 5 g Bacto-yeast extract, 10 g Bacto-tryptone (both products of DIFCO) and 10 g NaCl per liter, adjusted to pH 7.5. The antibiotic ampicillin was added to a final concentration of 50 μg/ml where indicated.

Escherichia coli strains were prepared for transformation by the calcium chloride procedure (Maniatis et al., supra, p. 254). The cells were transformed by the method of Dagert and Ehrlich [Gene 6, 23-28 (1979)].

A 200 μl sample of competent cells suspended in 50 mM CaCl₂, 20% glycerol was combined with 100 μl sample containing between 50 and 1000 ng of DNA in 10 mM CaCl₂. The mixture was kept on ice for 30 minutes and then heated at 42°C for 2 minutes. One ml of LB was added and the mixture was incubated at 37°C for 1 hour. The cells were plated on 37°C LB agar plates with ampicillin and incubated for 16 hours at 37°C to select for transformants.

COS cells were transfected using methods described by Butnick et al. [Mol. Cell. Biol. 5, 3009-3016 (1985)]. Ten-cm tissue culture dishes were seeded with 3 x 10⁶ COS cells in 10 ml of IMDM supplemented with 10% fetal calf serum (FCS), 1% fungizone (trade name of Grand Island Biological Company for Amphotericin B) and 50 μg/ml gentamycin and incubated overnight in a 37°C, 5% CO₂ humidified incubator. The cells were washed once with 37°C phosphate buffered saline (PBS), and 2 ml of 37°C PBS containing 1 mg/ml of DEAE-dextran (Pharmacia) and the DNA to be transfected was added to the cells. The COS cells were then incubated and gently shaken at 5-minute intervals over a 30 minute period.

After this incubation, 20 ml of IMEM (Iscove's Modified Eagle's Medium) containing 10% FCS, 2% fungizone, 50 μg/ml gentamycin and 100 μM chloroquine were added to each dish. After 2 1/2 hours of further incubation the medium was replaced with fresh IMEM medium with 10% FCS, 1% fungizone, 50 μg/ml gentamycin and 10% dimethylsulfoxide for 2.5 minutes. This medium was then replaced with IMDM containing 10% FCS 1% fungizone and 50 μg/ml gentamycin, the cultures were incubated at 37°C for 72 hours, and the cells and media were then analyzed as described below.

E. coli strain MC1061 is fresly available from the ATCC under accession No. ATCC 53338. This strain has also been described by Casadaban et al. [J. Mol. Biol. 138, 179-207 (1980)].

The COS cell line is freely available from the ATCC under accession No. CRL 1651. This cell line has also been described by Gluzman et al. [Cell 23, 175-182 (1981)].

Primer-directed site-specific mutagenesis was performed according to the methods described by Morinaga et al., supra. The synthetic oligonucleotide used to carry out the mutagenesis procedure was prepared by the phosphoramidite solid support method of Matteucci and Caruthers, supra.

Colony hybridization was performed using a method described by Maniatis et al., supra, p. 312. The same oligonucleotide used for primer-directed mutagenesis was used as a probe for the hybridizations after 5' end labeling with γ-[³²P]-ATP using polynucleotide kinase according to the procedure of Maniatis et al., supra, p. 396. The labeling of larger DNA probes used to locate the PLAP gene was carried out using a random priming kit (International Biotechnologies, Inc.) according to the manufacturer's instructions.

Enzymatic activity of SEAP was determined as described by McComb et al., supra, with p-nitrophenyl phosphate as substrate. The incubation mixture was made 10mM in L-homoarginine and heated for 10 minutes at 37°C prior to assay to inhibit endogeneous phosphatase activity. One unit of alkaline phosphatase activity corresponds to 1 μmol of substrate hydrolyzed per minute at 37°C.

One 10 cm plate of COS cells was set up and transfected as described above. Sixty hours post transfection, an aliquot of medium was transferred into a 1.5 ml Eppendorf tube. The medium was clarified by centrifugation for 2 minutes at 4°C at top speed. The clarified medium was removed to another Eppendorf tube and heated at 65°C for 5 minutes. The medium was centrifuged for 2 minutes at 25°C at top speed. The medium was removed to a new Eppendorf tube and kept at 4°C. The medium samples were assayed in two ways.

In the first assay, 10 μl of medium, 80 μl of alkaline phosphatase (AP) assay buffer (1.0 M Diethanolamine-HCl, pH 9.8, 0.5 mM MgCl2, 20 μM ZnSO4) and 10 μl of 100 mM L-homoarginine were preincubated for 10 minutes at 37°C in a 96-well flat bottom cell culture plate (Corning). Twenty μl of 60 mM p-nitrophenylphosphate in Ap assay buffer prewarmed to 37°C were then added to the above mixture. The OD405 of the reaction mixture was read in an Artek automatic vertical beam plate reader.

In the second assay, 100 μl of medium, 200 μl of 1.3 x AP assay buffer and 30 μl of 100 mM L-homoarginine were preincubated at 37°C. Twenty μl of 174 mM p-nitrophenyl phosphate in 1.3 x AP assay buffer prewarmed to 37°C were added, and the enzyme reaction was followed as above. The heating step and the inclusion of L-homoarginine in the assay mixture were performed to inhibit endogeneous phosphatase activities.

EXAMPLE 1 - PREPARATION OF HUMAN PLACENTAL ALKALINE PHOSPHATASE cDNA

A λgt10 library consisting of about 500,000 independent recombinant plaques was constructed according to the method of Huynh et al, in DNA Cloning: A Practical Approach, 1985, Glover, ed., IRL, Arlington, VA, Vol. 1, pp. 49-78, which is hereby incorporated by reference. The cDNA used was derived from placental polyadenylated mRNA, isolated from a single term placenta obtained from Mountainside Hospital, Montclair, NJ. The cDNA was prepared in vitro by the method of Gubler et al., Gene 25, 263-269 (1983), hereby incorporated by reference, and about 10 ng were used for construction of the library.

Screening was carried out by the tetramethylammonium chloride method [Wood et al., Proc. Natl. Acad. Sci. USA 82, 1585-1588 (1985)], using two oligodeoxyribonucleotides encompassing nucleotide regions 600-624 and 1495-1521 of the published sequence of human placental alkaline phosphatase (PLAP). The nucleotide sequence of one PLAP allele has been disclosed by Kam et al., supra.

Four independent clones were obtained, two of which contained a full-length insert for PLAP. Seventy-five percent of one of the inserts, designated PLAP 2.4, was sequenced by the method of Sanger et al., Proc. Natl. Acad. Sci. USA 74, 5463-5467 (1977), using a BRL gel sequencing system. This insert proved to be identical to the allele reported by Millán, J. Biol. Chem. 261, 3112-3115 (1986).

EXAMPLE 2 - CONSTRUCTION OF PLASMID pBC12/PLAP, ENCODING COMPLETE HUMAN PLACENTAL ALKALINE PHOSPHATASE

Insert PLAP 2.4 was excised from its clone using EcoRI and then blunt-ended with Klenow DNA polymerase I. The result was a blunt-ended 2.3 kilobase (kb) cDNA fragment containing the entire translated region and 0.7 kb of a 3'-untranslated region of human PLAP [Berger et al., Proc. Natl. Acad. Sci. USA 84, 695-698 (1987)].

To construct plasmid pBC12/PLAP, the 2.3 kb fragment was inserted into plasmid pBC12BI as described by Berger et al., Proc. Natl. Acad. Sci. USA 84, 4885-4889 (1987). Plasmid pBC12BI is a eukaryotic expression vector containing a bacterial origin of replication, a β-lactamase (ampicillin resistance, ampR) gene, a simian virus 40 origin of replication, the Rous sarcoma virus long terminal repeat transcription control region, and the genomic rat preproinsulin gene. The latter contributes an intron and an efficient polyadenylation signal.

The expression vector pBC12BI has been described [Cullen, Methods in Enzymology 152, 684-704 (1987)] and differs from the available starting vector pBC12MI only in that pBC12BI lacks a 74 base pair BstNI to HindIII fragment which was excised from the Rous Sarcoma Virus long terminal repeat present in pBC12MI. The absence or presence of this sequence has no effect on the utility of this promoter or vector. Plasmid pBC12BI in E. coli strain RR1 has been deposited with the American Type Culture Collection under the Budapest Treaty on February 4, 1988 and has been assigned accession No. ATCC 67617.

Plasmid pBC12/PLAP was used to construct the final exemplary expression vectors of this invention in several steps. First, a segment of the expression vector pBC12/PLAP was deleted. Secondly, the PLAP gene was mutated to encode a secreted form of the enzyme. Finally, the mutated gene was altered at both termini, so that it could be readily inserted into appropriately prepared expression vectors.

EXAMPLE 3- CONSTRUCTION OF SECRETED HUMAN PLACENTAL ALKALINE PHOSPHATASE EXPRESSION VECTOR pBC12/PLAPΔ489

PLAPΔ489 is the human placental alkaline phosphatase lacking the 24 carboxyl-terminal amino acid residues of the wild-type enzyme. This mutant was produced by placing a stop codon in the wild type PLAP gene after amino acid residue 489, by the site-directed mutagenesis method described above.

Before site-directed mutagenesis of the PLAP gene could be performed, a 700 nucleotide fragment of plasmid pBC12/PLAP was deleted by restriction endonuclease treatment with KpnI. Five $\mu$g of pBC12/PLAP DNA were incubated with 48 units of enzyme for 3 hours at 37°C. The reaction mixture was then run in a 0.9% agarose gel, and the cut vector was isolated as described above. One-hundred-fifty ng of this vector were incubated with T4 DNA ligase for 16 hours at 4°C, and the reaction mixture was used to transform E. coli strain MC1061 cells.

Transformants were selected for ampicillin resistance. Plasmid DNA was obtained by performing mini-lysates of several colonies and analyzing by KpnI cleavage followed by electrophoresis through a 1% agarose gel. A large scale plasmid preparation was made from cells containing the vector lacking the 700 nucleotide KpnI fragment. This plasmid was designated pBC12/PLAPΔKPN.

To carry out site-directed mutagenesis of pBC12/PLAPΔKPN, a 29-mer deoxyoligonucleotide primer was prepared by the phosphoramidite solid support method as described above. The sequence of this 29-mer primer and that of the region of the DNA to be mutated are shown in Fig. 1. The 29-mer primer contained 12 deoxynucleotides complementary to the bases on each side of the region to be mutated on one of the DNA strands. Mutation of the wild type gene to the 5 non-complementary nucleotides contained in the 29-mer primer (shown underlined in Fig. 1) created a stop codon after the nucleotides encoding amino acid residue 489 and a novel HpaI site in the resulting vector.

Twenty picomoles of the 29-mer primer were phosphorylated with 10 units of polynucleotide kinase (New England Biolabs) by incubating for 2 hours at 15°C in buffer containing 70 mM Tris-HCl, pH 7.6, 10 mM MgCl$_2$, 5 mM dithiothreitol and 100 mM ATP. One unit of activity is defined as the amount of enzyme catalyzing the production of one nmole of acid-insoluble $^{32}$P in 30 minutes at 37°C.

To carry out the mutagenesis procedure, 20 $\mu$g of pBC12/PLAPΔKPN were incubated with 36 units of BssHII and 84 units of KpnI in buffer containing 25 mM Tris-HCl, pH 7.8, 10 mM MgCl$_2$, 100 $\mu$g/ml bovine serum albumin and 2 mM $\beta$-mercaptoethanol for 24 hours at 37°C. The largest vector fragment produced, called the "gapped vector", was isolated in a 1% preparative agarose gel. Twenty $\mu$g of pBC12/PLAPΔKPN were linearized with 20 units of PvuI by incubating at 37°C for 16 hours, extracted with phenol/chloroform, precipitated in ethanol and taken up in water.

Equal 100 ng quantities of the linearized and gapped plasmids were mixed with a 20-fold molar excess of the phosphorylated oligonucleotide in 10 $\mu$l of water, and 2 $\mu$l of 10x Klenow buffer [1 M NaCl, 65 mM Tris-HCl (pH 7.4), 45 mM MgCl$_2$ and 10 mM 2-mercaptoethanol] were added. The mixture was treated for successive periods of 5, 30, 30 and 5 minutes at 100°C, room temperature, 4°C and on ice, respectively, after which the sample was brought to a volume of 20 $\mu$l by the addition of 2 $\mu$l of 10 mM ATP, 4 $\mu$l of a mixture of 2.5 mM each of dCTP, dATP dGTP, and TTP, 0.5 $\mu$l of Klenow polymerase I (2.5 units of activity) and 1 $\mu$l of T4 DNA ligase (0.8 units of activity).

The mixture was incubated for 16 hours at 15°C and then used directly to transform E. coli strain MC1061. Transformants were selected on LB agarose plates with ampicillin, and colonies from the plates were transferred onto nitrocellulose filters and screened for the presence of a sequence homologous to the 29-mer primer used in the mutagenesis procedure, using the synthetic oligonucleotide kinase labeled with $\gamma$-[$^{32}$P]-ATP as a probe.

Positive colonies were further screened by the method of Birnboim and Doly, supra, for the presence of an expected HpaI restriction site. Because colonies obtained by this procedure are mixed (Morinaga et al., supra), a positive DNA sample was used to retransform E. coli MC1061, and resulting colonies were rescreened for the HpaI restriction site. A positive colony thus identified, which was designated pBC12/PLAPΔ489, was identical to pBC12/PLAPΔKPN but for the 5 mutated nucleotides.

## EXAMPLE 4- CONSTRUCTION OF FINAL EXPRESSION VECTORS pBC12/RSV/SEAP AND pBC12/HIV/SEAP

The preparation of the final expression vectors was carried out in stages, entailing in succession (1) preparation of the vectors into which a modified human alkaline phosphatase gene could be inserted. (2) modification of the human alkaline phosphatase gene to obtain a moveable human alkaline phosphatase gene fragment, and (3) insertion of this fragment into the prepared vectors.

One of the plasmids used for these constructions was pBC12BI, described above. The other was pBC12/HIV/IL-2 [Cullen, Cell 46, 973-982 (1986)]. Plasmid pBC12/HIV/IL-2 was prepared by excising the

9

Rous Sarcoma Virus Long Terminal Repeat from the available vector pBC12/RSV/IL-2 by cleavage with NcoI and HindIII. Subsequently, a Human Immunodeficiency Virus (HIV) Long Terminal Repeat (LTR) XhoI to HindIII DNA fragment was inserted using standard techniques. Plasmid pBC12/HIV/IL-2 in E. coli strain RR1 has been deposited with the American Type Culture Collection under the Budapest Treaty on February 4, 1988 and has been assigned accession No. ATCC 67618.

To construct the final exemplary expression vectors of this invention, 5 μg of plasmid pBC12BI or pBC12/HIV/IL-2 DNA were treated with 20 units of BamHI in 50 μl of restriction enzyme buffer for 2 hours at 37° C. The reaction mixture was then blunt-ended by Klenow fragment treatment with 5 units of enzyme for 20 minutes at 25° C, after which the reaction was stopped by phenol extraction and ethanol precipitation.

One μg of blunt-ended pBC12BI or pBC12/HIV/IL-2 DNA was then incubated in 10 μl of ligation enzyme buffer together with 0.2 μg of phosphorylated XhoI linker DNA [d(pCCTCGAGG); New England Biolabs] and 1 unit of T4 DNA ligase at 15° C for 15 hours.

The reaction was stopped by ethanol precipitation, and the XhoI linker concatamers created by the ligation reaction were trimmed by treatment with 60 units of XhoI in 100 μl of restriction enzyme buffer for 3 hours at 37° C. After an additional ethanol precipitation step, the pBC12BI or pBC12/HIV/IL2 DNA was incubated for self-ligation in 20 μl of ligation enzyme buffer in the presence of 1 unit of T4 DNA ligase at 15° C for 15 hours. The ligation reaction mixture was then used directly to transform E. coli strain MC1061, and transformants were selected on LB agar with ampicillin.

The DNA obtained from ampicillin resistant colonies was screened by restriction endonuclease cleavage with XhoI, followed by analysis of the DNA fragments produced by electrophoresis in a 1% agarose gel containing 10 μg/ml ethidium bromide.

Plasmids derived from starting vector pBC12BI or pBC12/HIV/IL2, which had acquired a XhoI site, were thus prepared and identified. These plasmids were designated plasmid pBC12BI(+X) and plasmid pBC12/HIV/IL2(+X), respectively.

Plasmids pBC12BI(+X) and pBC12/HIV/IL2(+X) were then prepared in larger quantity by the lysis procedure of Birnboim and Doly, supra. Final preparation of the cloning vectors was carried out by cleaving 5 μg of pBC12BI(+X) or pBC12/HIV/IL2(+X) DNA with 20 units of HindIII and 20 units of XhoI, and isolating a 3.9 kb pBC12BI(+X) or 4.4 kb pBC12/HIV/IL2(+X) vector fragment after electrophoresis through a 1% agarose gel.

Having thus prepared the vectors used to receive a modified human alkaline phosphatase gene, the modified gene was prepared using plasmid pBC12/PLAPΔ489. To obtain a moveable human alkaline phosphate gene fragment for cloning into vectors pBC12BI(+X) and pBC/HIV/IL2(+X), unique restriction sites were created in pBC12/PLAPΔ489 at the 5' and 3' ends of the human alkaline phosphatase gene.

Five μg of plasmid pBC12/PLAPΔ489 DNA were treated with 20 units of ApaI in 50 μl of restriction enzyme buffer for 2 hours at 37° C. After ethanol precipitation, the DNA was blunt-ended by T4 DNA polymerase with 1 unit of enzyme for 5 minutes at 37° C, after which the reaction was stopped by phenol treatment and another ethanol precipitation.

One μg of blunt-ended pBC12/PLAPΔ489 DNA was then incubated together with 0.2 μg of HindIII linker DNA (phosphorylated 5'-GAAGCTTC-3') and 1 unit of T4 DNA ligase in 10 μl of ligation enzyme buffer at 15° C for 15 hours. After stopping the ligation reaction by ethanol precipitation, the excess HindIII linker DNA ligated to pBC12/PLAPΔ489 was removed by treatment with 60 units of HindIII in 100 μl of restriction enzyme buffer for 3 hours at 37° C.

After ethanol precipitation, the modified pBC12/PLAPΔ489 DNA was self-ligated in 20 μl of ligation reaction buffer with 1 unit of T4 DNA ligase at 15° C for 15 hours. The ligation reaction mixture was used directly to transform E. coli strain MC1061, and transformants were selected in LB agar containing ampicillin. The plasmid DNA of ampicillin resistant colonies was screened by restriction endonuclease cleavage with HindIII and ApaI, followed by gel electrophoresis in 1% agarose. One plasmid which had lost the ApaI site and acquired a HindIII site was thus identified and designated plasmid pBC12/PLAPΔ489(+H). A large-scale preparation of this plasmid was made as described above.

Five μg of plasmid pBC12/PLAPΔ489(+H) DNA were treated as noted above with 20 units of KpnI and then blunt-ended by T4 DNA polymerase. To 1 μg of this DNA, 0.2 μg of XhoI linker DNA was ligated as described above. After transformation of E. coli strain MC1061 with the ligation mixture and screening of the plasmid DNA of ampicillin resistant colonies with the restriction endonucleases XhoI and KpnI, one plasmid which had lost the KpnI site and acquired a XhoI site was identified and designated pBC12/PLAP(+H,X).

After these manipulations, the human alkaline phosphatase gene was flanked by unique HindIII and XhoI restriction sites in plasmid pBC12/PLAPΔ489(+H,X). A large-scale plasmid preparation of pBC12/PLAPΔ489(+H,X) was performed as above.

Final preparation of the human alkaline phosphatase gene fragment was carried out by cleaving 5 μg of

pBC12/PLAPΔ489(+H,X) with 20 units each of HindIII and XhoI. A 1.9 kb human alkaline phosphatase gene fragment was isolated after electrophoresis through a 1% agarose gel. This gene fragment was inserted into the above vectors as follows.

One hundred ng of the prepared vector fragments of pBC12BI(+X) or pBC12/HIV/IL2(+X) were mixed with 500 ng of the human alkaline phosphatase gene fragment in 20 μl of ligation buffer and incubated with 1 unit of T4 DNA ligase at 15° C for 15 hours.

The ligated DNA was then used to transform E. coli strain MC1061, and transformants were selected on LB agarose plates with ampicillin. The plasmid DNA of resistant colonies was screened by restriction endonuclease digestion with HindIII and XhoI, followed by 1% agarose gel electrophoresis. Two plasmids containing the appropriately modified human placental alkaline phosphatase gene thus prepared were identified and designated pBC12/RSV/SEAP and pBC12/HIV/SEAP (Fig. 2).

EXAMPLE 5 - COMPARISON OF THE USE OF THE SEAP INDICATOR GENE WITH OTHER INDICATOR GENES

To demonstrate the utility of the above plasmids containing the SEAP gene under the control of the RSV LTR or HIV LTR promoter, similar constructions were made containing the CAT and human IL-2 indicator genes.

Plasmid pBC12/RSV/IL2, containing a human IL-2 gene under the control of the RSV/LTR promoter, was constructed from vector pBC12MI as described by Cullen in U.K. patent application, publ. no. GB 2190 382A which is hereby incorporated by reference. Plasmid pBC12MI is identical to pBC12BI except that the LTR fragment contained therein extends an additional 74 bp in the 3' direction. Plasmid pBC12MI is freely available from the American Type Culture Collection, its accession No. being ATCC 67109.

The construction of pBC12/RSV/IL2 was carried out by treating 1 μg of pBC12MI DNA with 20 units of BamHI in 100 μl of restriction enzyme buffer for 1 hour at 37° C. The reaction mixture was then blunt-ended by Klenow fragment treatment with 4 units of enzyme for 2 hours at 15° C, after which the reaction was stopped by heating to 65° C for 5 minutes. Two μl of the mixture were then diluted 1:10 with ligation buffer, and the mixture was cooled on ice. One unit of T4 DNA ligase was added, and the reaction mixture was incubated for 16 hours at 4° C.

The ligation reaction mixture was then used directly to transform E. coli strain GM119 (freely available from the American Type Culture Collection under accession No. ATCC 53339), and transformants were selected in LB agar with ampicillin. The DNA from ampicillin resistant colonies thus obtained was screened by restriction endonuclease cleavage with BamHI or ClaI, followed by analysis of the DNA fragments produced by electrophoresis in a 1% agarose gel containing 10 μg/ml ethidium bromide. One plasmid which had lost the BamHI site but acquired a ClaI site in its place was thus identified and designated plasmid pBC12CI.

Plasmid pBC12CI was then prepared in larger quantity by the detergent lysis procedure of Clewell et al., supra. Final preparation of the cloning vector was carried out by cleaving 1 μg of pBC12CI DNA with 20 units of ClaI and blunt-ending the digestion product with Klenow fragment DNA polymerase I.

Plasmid pIL-2-2b, containing a cDNA copy of the entire 459 bp coding region of human IL-2 flanked by 31 bp of 5' non-translated sequence and 309 bp of 3' non-translated sequence [Taniguchi et al., Nature 302, 305-310 (1983)], was used as a source of the IL-2 gene for pBC12/RSV/IL2. The coloning of this IL-2 cDNA copy into plasmid pIL-2-2b has been described by Smith et al. [Proc. Natl. Acad. Sci. USA 82, 8404-8408 (1985)].

Ten μg of pIL-2-2b were cleaved with 20 units each of RsaI and BamHI in 100 μl of restriction enzyme buffer for 1 hour at 37° C. RsaI cleaves the IL-2 cDNA insert at a single site 1 bp 3' to the IL-2 initiation codon. The reaction mixture was then treated with Klenow fragment DNA polymerase I and subjected to preparative electrophoresis in a 1% low melting agarose gel. The desired 760 bp RsaI/BamHI IL-2 cDNA fragment was identified and extracted from the gel.

One hundred ng of the prepared vector pBC12CI were mixed with 100 ng of the RsaI/BamHI II-2 fragment in 30 μl of ligation buffer containing 2 units of T4 DNA ligase and incubated overnight at 4° C. The ligated DNA was then used to transform E. coli strain MC1061, and transformants were selected on LB agarose plates with ampicillin.

Two-hundred ng of the isolated IL-2 fragment were used to generate a [32P] labeled nick-translated probe using the Amersham nick translation kit according to the manufacturer's instructions. Colonies from the plates were transferred onto nitrocellulose filters (Schleicher and Schuell Inc.) and then screened for the presence of the IL-2 insert using the labeled probe and following the procedures of Maniatis et al., supra, page 324. Hybridization positive colonies were picked and DNA mini-preparations were made by the Birnboim and Doly procedure [supra]. These preparations were cleaved with HindIII and StuI and screened by gel electrophoresis for the presence of a characteristic 690 bp fragment which is indicative of the correct construction. This construction was designated pBC10.

A larger quantity of pBC10 DNA was prepared, and 10 μg were cleaved with 20 units of HindIII and 16 units of StuI in restriction enzyme buffer for 1 hour at 37° C. The resulting 690 bp IL-2 DNA fragment was isolated by preparative agarose gel electrophoresis.

One μg of plasmid pBC12MI DNA was cleaved with BamHI and then treated with Klenow DNA polymerase. After incubation at 65° C, the DNA was cleaved with HindIII, extracted with aqueous phenol-chloroform (proportions) and precipitated by the addition of a half-volume of 7.5 M ammonium acetate and 2 volumes of ethanol, followed by incubation at -70° C. The precipitated pBC12MI DNA was recovered by centrifugation washed with ethanol and resuspended in water.

One-hundred ng of the prepared pBC12MI vector was ligated to 200 ng of the isolated IL-2 HindIII/StuI fragment in 30 μl of ligation buffer, as previously described. The ligation mixture was used to transform E. coli strain MC1061, and transformants were selected on LB agarose plates with ampicillin. Individual colonies were screened as before for the presence of a 690 bp HindIII/BamHI fragment, and a construction meeting this criterion was designated pBC12/RSV/IL-2.

Plasmid pBC12/HIV/IL-2, containing a gene encoding human IL-2 under the control of the HIV LTR promoter, was prepared as described above.

Plasmid pBC12/RSV/CAT, containing a CAT gene under the control of the RSV LTR promoter, was prepared by replacing the IL-2 HindIII to BamHI fragment in pBC12/RSV/IL-2 with the HindIII to BamHI CAT coding fragment described by Gorman et al. [Mol. Cell. Biol. 2, 1044-1051 (1982)]. Similarly, pBC12/HIV/CAT was constructed from pBC12/HIV/IL-2 (see Fig. 2).

To make this comparison of the various indicator genes, it was necessary to test the genes under the control of the HIV LTR promoter both in the presence and absence of the tat gene product, which causes greatly elevated levels of HIV LTR-specific gene expression. Plasmid pBC12/CMV/t2, which expresses this gene product, was thus constructed as a source of the tat protein.

Plasmid pBC12/CMV/t2 was constructed as described by Cullen [Cell 46, 973-982 (1986)].

To demonstrate the expression of the SEAP gene product, COS cells were transfected as described above using plasmids pBC12/HIV/SEAP and pBC12/RSV/SEAP, with or without the co-transfected plasmid pBC12/CMV/t2 as a source of tat protein [see Cullen, Methods in Enzymology 152, 684-704 (1987); Cullen, Cell 46, 973-982 (1986)]. Aliquots of medium from the cells were then assayed for enzymatic activity as described above. Medium from COS cells harboring plasmid pBC12/HIV/CAT, prepared and assayed in the same way, was assayed as a control.

Aliquots were taken after 0, 1, 5, 10, 15, 20, 40 and 60 minutes of incubation and measured at 405 nm, with the results shown in Fig. 3.

Fig. 3 shows in panel A that 10 μl aliquots of medium from cells harboring plasmid pBC12/RSV/SEAP produced strong hydrolysis of p-nitrophenyl phosphate over time (closed triangles). Medium from cells harboring plasmid pBC12/HIV/SEAP was also highly active, but only when the tat gene product was present in the cultures (closed squares). When tat protein was not present in these cultures, no significant alkaline phosphate activity was present in the culture medium (open squares). Similarly there was no significant activity in the medium of cells transfected with plasmid pBC12/HIV/CAT even if the tat protein was present (open circles), showing the specificity of the SEAP assay and the absence of significant endogeneous alkaline phosphatase activity in the COS cells under the assay conditions.

Panel B shows higher activity levels, since 100 μl aliquots of medium were taken there for analysis. But again, the data show the specificity and sensitivity of the SEAP gene assay. Only a small amount of alkaline phosphatase activity is seen in the absence of tat protein.

To demonstrate the advantages of using the SEAP gene over the other indicator genes, SEAP activity was assayed as described above. IL-2 activity in the media of COS cultures transfected with plasmid pBC12/HIV/IL-2 or pBC12/RSV/IL-2 was assayed by the method of Gillis et al., J. Immunol. 120, 2027-2032 (1978). Activity in COS cells transfected with plasmid pBC12/HIV/CAT or pBC12/RSV/CAT was assayed, after lysing the cells by 3 cycles of freezing and thawing, by the method of Gorman et al., Mol. Cell. Biol. 2, 1044-1051 (1982). All of the indicator genes under the control of the HIV promoter were examined with and without the tat gene product, as described above. The results are shown in Table 1.

Table 1

| Comparison of the Levels of Expression of Different Indicator Genes | | | |
|---|---|---|---|
| | SEAP[a] | IL-2[b] | CAT[c] |
| RSV LTR | 275 | 3072 | 16.8 |
| HIV LTR (tat-) | 2.1 | 96 | 0.33 |
| HIV LTR (tat +) | 247 | 6144 | 19.7 |
| Blank | 0.1 | <4 | <0.1 |

[a]SEAP activity is given in milliunits of alkaline phosphatase activity per ml of cell culture medium.

[b]IL-2 activity is given in units per ml of cell culture medium.

[c]CAT activity is given in percent conversion of chloramphenicol to its acetylated form in 30 minutes at 37° C, using a 1:100 dilution of the cell extract.

The data of Table 1 show that all three of the indicator genes examined produced high levels of activity that were far above background levels. The expression of genes under the control of the HIV LTR promoter was strongly dependent upon the presence of the tat gene product, as expected. On the basis of the ratios of activity in stimulated ( + tat) to unstimulated (-tat) cultures, the SEAP gene performed at the same level as the IL-2 and CAT genes; these ratios for SEAP, IL-2 and CAT were 118, 64 and 60, respectively. But on the basis of ease of analysis, the SEAP gene was markedly superior.

Compared to the commonly used CAT indicator gene, the use of the SEAP gene requires fewer assay steps and smaller amounts of reagents. Other advantages of the SEAP gene are summarized in Table 2.

Table 2

| Comparison of the SEAP and CAT Indicator Genes | | |
|---|---|---|
| Assay Aspect | SEAP | CAT |
| Radioisotopes | not used | used |
| Cell lysis | not required | required |
| Quantitation | fast and accurate, by ELISA readers, etc. | generally by densitometry, which measures only large changes; quantitation by liquid scintillation spectrometry requires scraping TLC plates |
| Assay time | results in minutes to 2 hours | requires 3 days |

Although the product of the IL-2 gene is also secreted in COS cell cultures, the assay required to detect IL-2 is a laborious and far more inaccurate cell culture bioassay.

Example 6- IMMUNOPRECIPITATION OF $^{35}$S-PULSE LABELED SEAP

To determine whether the increase in the steady state level of SEAP activity at 72 hours post-infection shown in Table 1 with and without tat protein was a reflection of an increased rate of SEAP protein production or an effect on the activity levels of existing enzyme molecules or enzyme turnover, the following experiment was carried out.

Thirty-five mm culture plates were seeded with $3 \times 10^5$ COS cells and transfected with plasmid pBC12/HIV/CAT or plasmid pBC12/HIV/SEAP as described above, in 2 ml of Iscove's Modified Dulbecco's Medium (IMDM). At 60 hours post-transfection, the tissue culture medium was aspirated off and replaced with 1 ml of methionine-free MEM medium (GIBCO). The cells were preincubated for 1 hour at 37°C. The medium was aspirated off and replaced with 1 ml of methionine-free MEM medium containing 200 $\mu$Ci of $^{35}$S-methionine (Amersham 800 Ci/$\mu$mol) and incubated at 37°C for 15 minutes. The pulse labeling was done in the presence and absence of tat as described above.

The medium was removed, and cells were harvested by the addition of 1 ml of room temperature radioimmunoprecipitation assay (RIPA) buffer (0.1% SDS, 1% Triton X-100, 1% sodium deoxycholate, 0.15 M NaCl, 0.01 M Tris-HCl, pH 7.4) and incubated for 5 minutes with swirling. The monolayer was then scraped, and the resulting suspension was transferred to a 1.5 ml Eppendorf tube, which was centrifuged in a microfuge for 15 minutes at 4°C at full speed to clarify the lysates.

The clarified lysates were transferred to another Eppendorf tube and incubated with 1 $\mu$l of antigen-affinity purified rabbit anti-human placental alkaline phosphatase antibodies (DAKO, Santa Barbara, CA) for 16 hours at 4°C. Two-hundred $\mu$l of a 2:1 suspension of Staphyloccus aureus protein A (Staph A, Calbiochem, La Jolla, CA) were added, and the mixture was incubated for 20 minutes at 4°C. The Staph A was pelleted by centrifuging 15 seconds in a microfuge. The supernatant fluid was removed and discarded.

The pellet was resuspended in 1 ml of RIPA buffer by vortexing. The pellet was washed twice more by this procedure. Twenty $\mu$l of 2x dissociation buffer (0.225 M Tris-HCl pH 6.8, 4% SDS, 20% glycerol, 10% $\beta$-mercaptoethanol, 0.2% bromophenol blue) were added to the final Staph A pellet. The immune complexes were dissociated by heating at 100°C for 2 minutes. The Staph A was pelleted by centrifugation, and the labeled material was resolved by electrophoresis in discontinuous SDS-polyacrylamide gel containing a 9% separating gel as described by Laemmli, Nature 227, 680-685 (1970). The gel was prepared for fluorography and exposed to pre-flashed X-ray film at -70°C with an intensifying screen, with the results shown in Fig. 4.

In Fig. 4 lanes 1 to 3 contained plasmids pBC12/HIV/CAT (tat+), pBC12/HIV/SEAP (tat-) and pBC12/HIV/SEAP (tat+), respectively. Lane M contained molecular weight marker proteins labeled with $^{14}$C. As can be seen, no immunoprecipitable protein was produced by plasmid pBC12/HIV/CAT (tat+) and only a small amount of protein was produced by plasmid pBC12/HIV/SEAP in the absence of tat. Strong production of SEAP protein was seen for plasmid pBC12/HIV/SEAP in the presence of tat. Thus, the difference in the level of expression with and without tat detected in this way is similar to the difference in activity measured in the cell supernatant in Table 1.

Many modifications and variations of this invention may be made without departing from its spirit and scope, as will become apparent to those skilled in the art. The specific embodiments described herein are offered by way of example only.

## Claims

1. A secretable human placental alkaline phosphatase having from 11 to 25 amino acid residues deleted at the C-terminus compared to the wild-type human placental alkaline phosphatase.

2. A secretable human placental alkaline phosphatase as claimed in claim 1 having 24 amino acid residues deleted at the C-terminus compared to the wild-type human placental alkaline phosphatase.

3. A DNA sequence coding for a secretable human placental alkaline phosphatase as claimed in claim 1 or 2.

4. A recombinant vector comprising a DNA sequence as claimed in claim 3 operatively linked to an expression control sequence.

5. A mammalian cell containing a recombinant vector as claimed in claim 4.

6. A process for producing a secretable human placental alkaline phosphatase as claimed in claim 1 or 2, comprising
(a) culturing a mammalian cell containing a recombinant vector as claimed in claim 4; and
(b) isolating the secretable human placental alkaline phosphatase from the culture.

7. A process according to claim 6 in which the recombinant vector is introduced into the mammalian cell by transfection.

8. A process for detecting factors which influence gene expression, comprising:

(a) providing mammalian cells in culture containing a DNA sequence coding for a secretable human placental alkaline phosphatase as claimed in claim 1 or 2;

(b) contacting the mammalian cell culture with a factor influencing gene expression; and

(c) measuring the amount of secretable human placental alkaline phosphatase in the culture medium.

9. The process of claim 8 in which the factor influencing gene expression is tat protein.

10. The use of a secretable human placental alkaline phosphatase as claimed in claim 1 or 2 for detecting factors which influence gene expression.

## Figure 1

PLAP            5' CGCGCACCCGGGGCGGTCCGTGGTCCCCG 3'

Oligonucleotide    3' GCGCGTGGGCCC<u>AATTG</u>GGCACCAGGGGC 5'

## Figure 2

## Figure 3

Figure 4

| DOCUMENTS CONSIDERED TO BE RELEVANT | | | EP 89101792.3 |
|---|---|---|---|
| Category | Citation of document with indication, where appropriate, of relevant passages | Relevant to claim | CLASSIFICATION OF THE APPLICATION (Int. Cl.4) |
| D,A | PROCEEDINGS OF THE NATIONAL ACADEMY OF SCIENCES OF THE STATES OF AMERICA, vol. 82, no. 24, December 1985, Baltimore, USA<br><br>W.KAM et al. "Cloning, sequencing, and chromosomal localization of human term placental alkine phosphatase cDNA"<br>pages 8715-8719<br><br>* Totality *<br><br>-- | 1-4 | C 12 N 15/00<br>C 12 N 9/24<br>C 12 N 5/00<br>C 12 Q 1/42 |
| D,A | CHEMICAL ABSTRACTS, vol. 104, no. 17, April 28, 1986, Columbus, Ohio, USA<br><br>J.L.MILLAN et al. "Molecular cloning and sequence analysis of human placental alkine phosphatase"<br>page 166, left column, abstract-no. 143 134y<br><br>& D.Biol.Chem. 1986, 261(7), 3112-12<br><br>---- | 1-4 | |
| | | | TECHNICAL FIELDS SEARCHED (Int. Cl.4) |
| | | | C 12 N<br>C 12 Q |

The present search report has been drawn up for all claims

| Place of search | Date of completion of the search | Examiner |
|---|---|---|
| VIENNA | 08-05-1989 | WOLF |